(19) Europäisches Patentamt
European Patent Office
Office européen des brevets

(11) Veröffentlichungsnummer: **0 056 969**
**B1**

(12) **EUROPÄISCHE PATENTSCHRIFT**

(45) Veröffentlichungstag der Patentschrift: **06.11.85**

(21) Anmeldenummer: **82100319.1**

(22) Anmeldetag: **18.01.82**

(51) Int. Cl.⁴: **C 07 D 239/42**, C 07 D 239/46, C 07 D 251/16, C 07 D 251/46, A 01 N 47/36

(54) **Substituierte Phenylsulfonylharnstoff-Derivate, Verfahren und neue Zwischenprodukte zu deren Herstellung sowie diese Derivate als Wirkstoffe enthaltende herbizide Mittel.**

(30) Priorität: **26.01.81 JP 8977/81**
**20.06.81 JP 94571/81**

(43) Veröffentlichungstag der Anmeldung: **04.08.82 Patentblatt 82/31**

(45) Bekanntmachung des Hinweises auf die Patenterteilung: **06.11.85 Patentblatt 85/45**

(84) Benannte Vertragsstaaten: **AT BE CH DE FR GB IT LI NL SE**

(56) Entgegenhaltungen:
**EP - A - 0 044 211**
**US - A - 4 127 405**
**US - A - 4 169 719**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Aya, Masahiro, 2-844, Suzuki-cho, Kodaira-shi Tokyo (JP)**
Erfinder: **Saito, Junichi, 3-7-12, Osawa, Mitaka-shi Tokyo (JP)**
Erfinder: **Yasui, Kazuomi, 7-6-15, Shakujii-dai, Nerima-ku Tokyo (JP)**
Erfinder: **Shiokawa, Kozo, 210-6, Shukugawara Tama-ku, Kawasaki-shi Kanagawa-ken (JP)**
Erfinder: **Morishima, Norihisa, 4-32-5, Owada-cho, Hachioji-shi Tokyo (JP)**
Erfinder: **Goto, Toshio, 5-20-1-304, Seishin, Sagamihara-shi Kanagawa-ken (JP)**

(74) Vertreter: **Gremm, Joachim, Dr. et al, Bayer AG c/o Zentralbereich Patente, Marken und Lizenzen, D-5090 Leverkusen 1, Bayerwerk (DE)**

EP 0 056 969 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue substituierte Phenylsulfonylharnstoff-Derivate, ein Verfahren und neue Zwischenprodukte zu deren Herstellung sowie herbizide Mittel, die diese Derivate als Wirkstoffe enthalten.

Im besonderen sind die neuen substituierten Phenylsulfonylharnstoff-Derivate gemäss der vorliegenden Erfindung Verbindungen der Formel

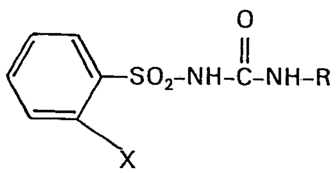
(I)

in der X für eine Phenyl-Gruppe oder eine Phenoxy-Gruppe und R für die Gruppe

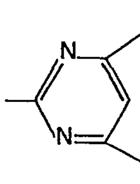 oder 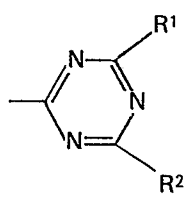

stehen, in denen $R^1$ und $R^2$ eine Methyl-Gruppe oder eine Methoxy-Gruppe bezeichnen.

Die substituierten Phenylsulfonylharnstoff-Derivate der vorstehenden Formel (I) können nach dem folgenden Verfahren hergestellt werden, und dementsprechend betrifft die vorliegende Erfindung auch ein Verfahren zu deren Herstellung, das heisst, ein Verfahren zur Herstellung substituierter Phenylsulfonylharnstoff-Derivate der vorstehenden Formel (I), das dadurch gekennzeichnet ist, dass eine Verbindung der Formel

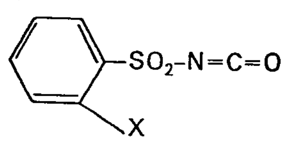
(II)

in der X die vorstehend genannte Bedeutung hat, mit einer Verbindung

$NH_2$-R (III)

in der R die vorstehend genannte Bedeutung hat, umgesetzt wird.

Ferner betrifft die vorliegende Erfindung auch herbizide Mittel, die substituierte Phenylsulfonylharnstoff-Derivate der vorstehenden Formel (I) als Wirkstoffe enthalten.

In den US-PSen 4 127 405 und 4 169 719 sowie in der japanischen Offenlegungsschrift 52-122 384 wurde beschrieben, dass Verbindungen der Formel

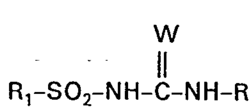
(IV)

in der R für

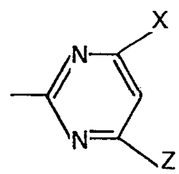 oder 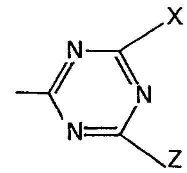

$R^1$ für

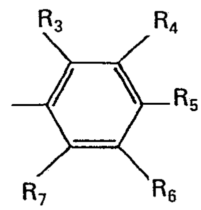 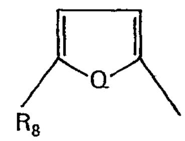

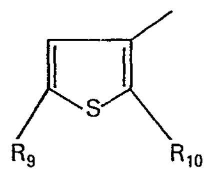 oder 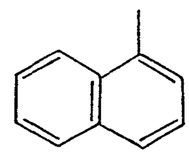 ;

$R_3$ und $R_6$ unabhängig voneinander für Wasserstoff, Fluor, Brom, Iod, Alkyl mit 1 bis 4 C-Atomen, Alkoxy mit 1 bis 4 C-Atomen, Nitro, Trifluormethyl, Cyano, $CH_3S(O)_n$– oder $CH_3CH_2S(O)_n$–, $R_4$ für Wasserstoff, Fluor, Chlor, Brom oder Methyl, $R_5$ für Wasserstoff, Fluor, Chlor, Brom, Methyl oder Methoxy, $R_7$ für Wasserstoff, Fluor, Brom, Alkyl mit 1–2 C-Atomen oder Alkoxy mit 1–2 C-Atomen, $R_8$ für Wasserstoff, Methyl, Chlor oder Brom, $R_9$ und $R_{10}$ unabhängig voneinander für Wasserstoff, Methyl, Chlor oder Brom, W und Q unabhängig voneinander für Sauerstoff oder Schwefel, n für 0, 1 oder 2, X für Wasserstoff, Chlor, Brom, Methyl, Ethyl, Alkoxy mit 1-3 C-Atomen, Trifluormethyl, $CH_3S$– oder $CH_3OCH_2$– und Z für Methyl oder Methoxy stehen, jedoch unter den Bedingungen, dass

a) für den Fall, dass $R_5$ nicht Wasserstoff ist, mindestens eine der Gruppen $R_3$, $R_4$, $R_6$ und $R_7$ nicht Wasserstoff sein darf und mindestens zwei der Gruppen $R_3$, $R_4$, $R_6$ und $R_7$ Wasserstoff sein müssen;

b) für den Fall, dass $R_5$ Wasserstoff ist und sämtliche der Gruppen $R_3$, $R_4$, $R_6$ und $R_7$ von Wasserstoff verschieden sind, sämtliche der Gruppen $R_3$, $R_4$, $R_6$ und $R_7$ entweder Chlor oder Methyl sein müssen; und

c) für den Fall, dass sowohl $R_3$ als auch $R_7$ Wasserstoff sind, mindestens eine der Gruppen $R_3$, $R_4$, $R_5$ und $R_6$ Wasserstoff sein muss; sowie landwirtschaftlich unbedenkliche Salze dieser Verbindungen herbizide Wirkung besitzen.

Als Ergebnis ausgedehnter Untersuchungen substituierter Phenylsulfonylharnstoff-Derivate, die der Suche nach solchen neuartigen Derivaten galten, die eine überlegene herbizide Wirkung besitzen, wurde nunmehr gefunden, dass die

neuen substituierten Phenylsulfonylharnstoff-Derivate der Formel (I) eine ausgezeichnete selektive herbizide Wirkung zeigen, die von den Verbindungen der vorstehenden Formel (IV) nicht erwartet werden kann.

Mit anderen Worten, im Vergleich mit den üblichen Herbiziden, die trotz ihrer herbiziden Wirkung bei verhältnismässig niedrigen Dosierungen bereits eine beträchtliche Phytotoxizität gegenüber Reispflanzen zeigen, zeigen die substituierten Phenylsulfonylharnstoff-Derivate gemäss der vorliegenden Erfindung überhaupt keine Phytotoxizität gegenüber Reispflanzen und üben schon bei niedrigen Dosierungen eine treffsichere, hervorragende, selektive herbizide Wirkung aus.

Die Verbindungen gemäss der vorliegenden Erfindung zeigen eine überlegene selektive Bekämpfungswirkung, insbesondere bei Verwendung als Bodenbehandlungsmittel vor dem Auflaufen und als Behandlungsmittel für Pflanzenstiele und Blätter sowie Bodenbehandlungsmittel für Unkräuter in Reisanpflanzungen. Die Verbindungen gemäss der vorliegenden Erfindung sind hervorragend in bezug auf Sicherheit, zeigen eine überlegene herbizide Wirkung und besitzen ein breites herbizides Spektrum. Sie sind beispielsweise dadurch gekennzeichnet, dass sie herbizide Aktivität gegen die nachstehend genannten Reisfeld-Unkräuter zeigen, ohne dass sie irgendeine schädliche Wirkung auf Reispflanzen ausüben:

Dikotyledonen:
Rotala indica Koehne
Lindernia procumbens Philcox
Ludwiga prostrata Roxburgh
Potamogeton distinctus A. Benn.
Elatine triandra Schk.

Monokotyledonen:
Echinochloa crus-galli Beauv. var.
Monochoria vaginalis Presl.
Eleocharis acicularis L.
Eleocharis Kuroguwai Ohwi
Cyperus difformis L.
Cyperus serotinus Rottboell
Sagittaria pygmaea Miq.
Alisma canaliculatum A. Br. et Bouche
Scirpus juncoides Roxburgh var. etc.

Sie sind ferner dadurch gekennzeichnet, dass sie herbizide Aktivität gegen solche Feld-Unkräuter wie nachstehend aufgeführt zeigen, ohne dass sie irgendeine schädliche Wirkung auf die nachstehend genannten Nutzpflanzen ausüben:

Dikotyledonen:
Polygonum sp.
Chenopodium album Linnaeus
Stellaria media Villars
Portulaca oleracea Linnaeus

Monokotyledonen:
Echinochloa crus-galli Beauv. var.
Digitaria adscendens Henr.
Cyperus iria L. etc.

Nutzpflanzen:
Dikotyledonen wie Senf, Senfpflanzen, Baumwolle, Möhren, Erbsen und Bohnen, Kartoffeln, Rüben, Kanaris etc.; Monokotyledonen wie Mais, Reispflanzen, Hafer, Gerste, Weizen, Hirse, Zukkerrohr etc.

Die vorstehend angegebenen Pflanzennamen sollten jedoch lediglich als Vertreter der durch den lateinischen Namen bezeichneten Gattungen angesehen werden. Die Anwendbarkeit der Wirkstoffe gemäss der vorliegenden Erfindung ist jedoch nicht auf Unkräuter in Reisfeldern und Feldern beschränkt, sondern sie sind auch gegenüber Unkräutern wirksam, die schädlich gegenüber Binsen etc. sind, Unkräutern auf Brachland und dergleichen. Der hier verwendete Begriff «Unkräuter» bezeichnet im weitesten Sinne sämtliche Unkräuter, die an Stellen wachsen, an denen sie nicht erwünscht sind.

Die substituierten Phenylsulfonylharnstoff-Derivate der vorgenannten Formel (I) gemäss der vorliegenden Erfindung werden nach dem folgenden allgemeinen Verfahren hergestellt.

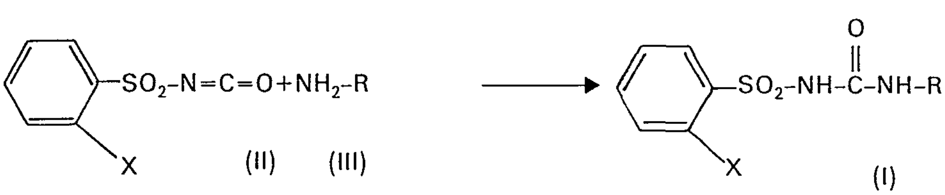

(II) (III) (I)

worin X und R die vorstehend genannten Bedeutungen haben. In dem vorstehenden Reaktionsschema sind spezielle Beispiele für X eine Phenyl-Gruppe und eine Phenoxy-Gruppe; spezielle Beispiele für R sind 4,6-Dimethylpyrimidin-2-yl, 4,6-Dimethoxypyrimidin-2-yl, 4-Methoxy-6-methylpyrimidin-2-yl, 4,6-Dimethyl-1,3,5-triazin-2-yl, 4,6-Dimethoxy-1,3,5-triazin-2-yl, 4-Methoxy-6-methyl-1,3,5-triazin-2-yl und ähnliche Gruppen. Bei dem Verfahren zur Herstellung der Verbindungen der vorgenannten Formel (I), das durch das vorstehende Reaktionsschema dargestellt wird, sind besondere Beispiele des einen Ausgangsmaterials, d.h. der Verbindung der Formel (II):

2-Biphenylsulfonyl-isocyanat und
2-Phenoxyphenylsulfonyl-isocyanat.

Die Zwischenprodukte der Formel (II) sind noch nicht bekannt. Sie können nach einem nicht zum Stand der Technik gehörenden, erfinderischen Verfahren hergestellt werden, indem man Phenylsulfonamide der Formel

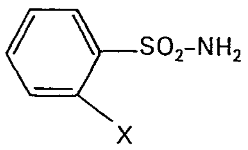

(V),

worin X wie oben angegeben für Phenyl oder Phenoxy steht, in Gegenwart eines aliphatischen Isocyanats und gegebenenfalls in Gegenwart eines Verdünnungsmittels mit Phosgen ($COCl_2$) oder Chlorameisensäure-trichlormethylester ($CCl_3O-CO-Cl$) umsetzt.

Verwendet man 2-Phenoxy-phenylsulfonamid und Phosgen als Ausgangsstoffe und n-Butylisocyanat als aliphatisches Isocyanat, so kann der Reaktionsablauf zusammenfassend durch folgendes Formelschema wiedergegeben werden:

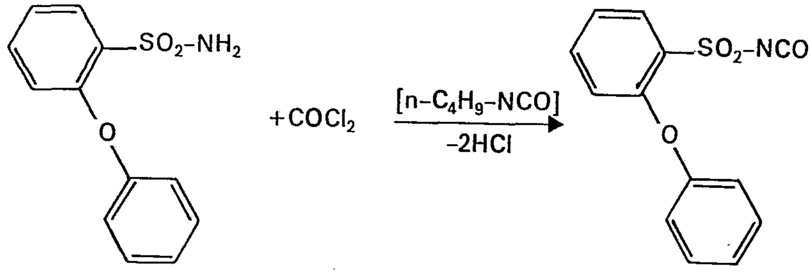


Es ist notwendig, dass diese Umsetzung in Gegenwart eines aliphatischen Isocyanats durchgeführt wird; dieses kann nach Beendigung der Reaktion wiedergewonnen und neu eingesetzt werden. Bevorzugt verwendet man Alkylisocyanate mit 3–8 C-Atomen oder Cycloalkylisocyanate mit 5–8 C-Atomen. Als Beispiele hierfür seien im einzelnen genannt: n-Butylisocyanat, n-Hexylisocyanat und Cyclohexylisocyanat.

Als Verdünnungsmittel für dieses Verfahren kommen alle üblichen inerten Lösungsmittel in Frage, beispielsweise Kohlenwasserstoffe wie Hexan, Cyclohexan, Petroläther, Ligroin, Toluol, Xylol; chlorierte Kohlenwasserstoffe wie z.B. Methylenchlorid, Chloroform, Tetrachlorkohlenstoff, Ethylenchlorid, Trichlorethylen oder Chlorbenzol; Äther wie Diethyläther, Methylethyläther, Di-isopropyläther, Dibutyläther, Dioxan, Tetrahydrofuran; Ketone wie z.B. Aceton, Methylethylketon, Methylisopropylketon oder Methylisobutylketon; Nitrile, wie z.B. Acetonitril oder Propionitril; Ester wie z.B. Essigsäureethyl- oder -amylester; Amide wie z.B. Dimethylformamid oder Dimethylacetamid; Sulfone und Sulfoxide wie z.B. Sulfolan und Dimethylsulfoxid; ferner Basen wie z.B. Pyridin.

Es ist vorteilhaft, die Umsetzung (V) → (II) in Gegenwart eines geeigneten Katalysators durchzuführen. Als geeignete Katalysatoren sind insbesondere tertiäre Amine zu nennen, wie z.B. Triethylamin, Dimethylcyclohexylamin und 1,4-Diazabicyclo(2.2.2)-octan.

Die Reaktionstemperaturen können bei der Umsetzung (V) → (II) in einem weiten Bereich variiert werden. Im allgemeinen arbeitet man bei Temperaturen zwischen -20°C und dem Siedepunkt des Reaktionsgemisches, vorzugsweise zwischen 80 und 150°C. Die Umsetzung wird im allgemeinen bei Normaldruck durchgeführt, sie kann jedoch auch bei erhöhtem oder vermindertem Druck durchgeführt werden.

Bei der Durchführung der Umsetzung (V) → (II) setzt man im allgemeinen auf 1 Mol Phenylsulfonamid (V) 1–2 Mol, vorzugsweise 1,05–1,3 Mol des aliphatischen Isocyanats und 1–4 Mol, vorzugsweise 1–1,5 Mol Phosgen bzw. 0,5–1 Mol, vorzugsweise 0,55–0,65 Mol Chlorameisensäure-trichlormethylester ein. Der Katalysator wird in Mengen von 0,1–5 g, vorzugsweise von 0,8–2,2 g pro Mol (V) eingesetzt.

Die Aufarbeitung und Isolierung der Phenylsulfonylisocyanate (II) erfolgt in üblicher Weise; diese Isocyxanate können beispielsweise durch Destillation gereinigt werden.

Das Herstellungsverfahren (V) → (II) wird durch die nachfolgenden Synthesebeispiele weiter erläutert:

Synthesebeispiel 1

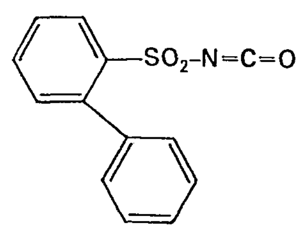

23,3 g 2-Biphenylylsulfonamid, 10,9 g n-Butylisocyanat, eine katalytische Menge 1,4-Diazabicyclo [2.2.2]octan und 150 ml Xylol wurden unter Rühren eine Stunde unter Rückfluss erhitzt. Dann wurde im Verlauf von 2,5 Stunden eine Lösung von 11,9 g Trichloromethyl-Chloroformiat in 30 ml Xylol tropfenweise hinzugefügt, wobei die Innentemperatur bei 120–125°C gehalten wurde. Nach der Zugabe wurde die gleiche Temperatur noch einige Zeit aufrechterhalten und danach der Inhalt zur Vervollständigung der Reaktion noch kurze Zeit zum Rückfluss erhitzt. Nach Beendigung der Reaktion wurden Xylol und n-Butylisocyanat unter vermindertem Druck abdestilliert, wonach 22,0 g des gewünschten 2-Biphenylylsulfonylisocyanats von hoher Reinheit erhalten wurden.
Sdp. 135–237°C/0,93–1,06 mbar (0,7–0,8 mmHg)

Synthesebeispiel 2

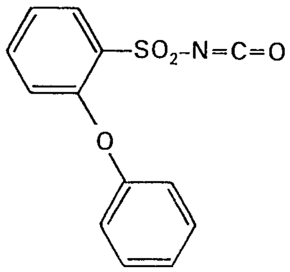

25,0 g 2-Phenoxyphenylsulfonamid, 14,0 g n-Hexylisocyanat, eine katalytische Menge 1,4-Diazabicyclo [2.2.2]octan und 150 ml Xylol wurden unter Rühren eine Stunde unter Rückfluss erhitzt. Danach wurde in ähnlicher Weise wie in Synthesebeispiel 1 mit einer Lösung von 11,9 g Trichloromethyl-Chloroformiat in 30 ml Xylol umgesetzt, und nach Beendigung der Reaktion wurden Xylol und n-Hexylisocyanat abdestilliert, wonach 22,8 g des gewünschten 2-Phenoxyphenylsulfonylisocyanat erhalten wurden.
Sdp. 146–150°C/0,93 mbar (0,7 mmHg).

Synthesebeispiel 3

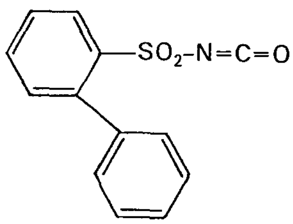

23,3 g 2-Biphenylylsulfonamid, 10,9 g n-Butylisocyanat, eine katalytische Menge 1,4-Diazabicyclo [2.2.2]octan und 180 ml Chlorbenzol wurden unter Rühren 2 Stunden unter Rückfluss erhitzt. Dann wurde im Verlauf von zwei Stunden Phosgen in einem geringen Überschuss über die theoretische Menge durch die Reaktionsmischung geleitet, wobei die Innentemperatur bei 115–120°C gehalten wurde. Soweit während dieser Zeit unumgesetztes Phosgen aus dem System entwich, wurde dieses in einer Trockeneis-Kühlfalle aufgefangen und in das Reaktionsgefäss zurückgeführt. Nach dem Einleiten des Phosgens wurde die Reaktionsmischung zur Vervollständigung der Reaktion kurze Zeit weiter bei Rückflusstemperatur gerührt. Der Inhalt wurde abgekühlt, erforderlichenfalls zur Entfernung unlöslicher Anteile unter vermindertem Druck filtriert und unter vermindertem Druck eingeengt, wonach 20,0 g des gewünschten 2-Biphenylylsylfonylisocyanats erhalten wurde.
Sdp. 135–137°C/0,93–1,06 mbar (0,7–0,8 mmHg).

Besondere Beispiele des anderen Ausgangsmaterials, d.h. der Verbindung der Formel (III), sind:

2-Amino-4,6-dimethylpyrimidin,
2-Amino-4-methoxy-6-methylpyrimidin,
2-Amino-5-methoxy-6-methyl-1,3,5-triazin,
2-Amino-4,6-dimethoxy-1,3,5-triazin etc.

Das erfindungsgemässe Verfahren, (II)+(III) → (I), wird durch das folgende repräsentative Beispiel im einzelnen erläutert.

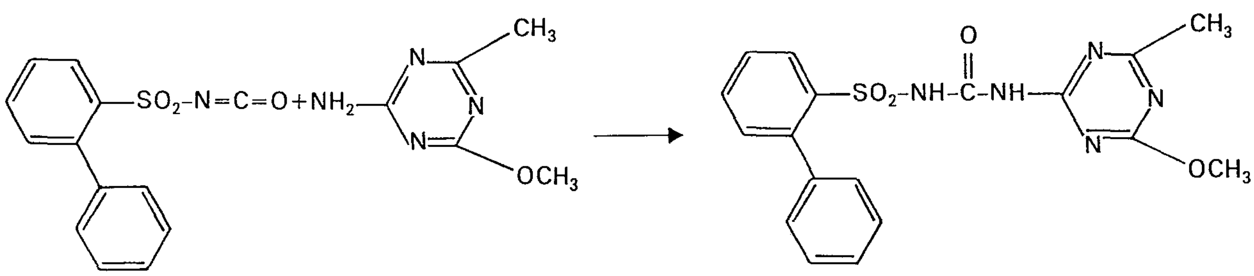

Das Verfahren zur Herstellung der Verbindungen gemäss der Erfindung wird vorzugsweise in Anwesenheit eines Lösungsmittels oder Verdünnungsmittels durchgeführt. Für diesen Zweck kann jedes inerte Lösungsmittel oder Verdünnungsmittel verwendet werden. Beispiele für solche Lösungsmittel oder Verdünnungsmittel sind aliphatische, alicyclische und aromatische Kohlenwasserstoffe (die gegebenenfalls chloriert sein können) wie Hexan, Cyclohexan, Petroläther, Ligroin, Benzol, Toluol, Xylol, Methylenchlorid, Chloroform, Kohlenstofftetrachlorid, Ethylenchlorid, Trichlorethylen, Chlorbenzol etc.; Äther wie Diethyläther, Methylethyläther, Di-isopropyläther, Dibutyläther, Propylenoxid, Dioxan, Tetrahydrofuran etc.; Ketone wie Aceton, Methylethylketon, Methyl-isopropylketon, Methyl-isobutylketon etc.; Nitrile wie Acetonitril, Propionitril, Acrylnitril etc.; Ester wie Ethylacetat, Amylacetat etc.; Säureamide wie Dimethylformamid, Dimethylacetamid etc.; Sulfone, Sulfoxide etc. wie Dimethylsufloxid, Sulfolan etc.; und Basen wie Pyridin etc.

Das Verfahren gemäss der vorliegenden Erfindung kann wirksam in Gegenwart eines Katalysators durchgeführt werden; Beispiele hierfür sind 1,4-Diazabicyclo [2.2.2]octan etc.

Das Verfahren gemäss der Erfindung kann über einen weiten Bereich von Temperaturen durchgeführt werden. Im allgemeinen wird es bei einer Temperatur zwischen -20°C und dem Siedepunkt des Reaktionsgemisches, vorzugsweise zwischen 0°C und 100°C, durchgeführt. Die Reaktion wird vorzugsweise unter Umgebungsdruck durchgeführt, jedoch kann auch unter erhöhtem oder vermindertem Druck gearbeitet werden.

Wenn die Verbindungen gemäss der vorliegenden Erfindung als Herbizide eingesetzt werden,

können sie als solche nach einfacher Verdünnung mit Wasser oder aber in Form verschiedenartiger landwirtschaftlicher Präparate eingesetzt werden, die unter Verwendung in der Landwirtschaft gebräuchlicher Hilfsstoffe, entsprechend den üblichen Verfahrensweisen bei der Herstellung von Agrochemikalien, hergestellt werden.

Diese verschiedenartigen Präparate können beim praktischen Gebrauch als solche verwendet werden, oder sie können mit Wasser auf eine gewünschte Konzentration verdünnt werden. Der hierin verwendete Begriff «in der Landwirtschaft gebräuchliche Hilfsstoffe» umfasst beispielsweise Verdünnungsmittel (Lösungsmittel, Füllstoffe und Träger), oberflächenaktive Mittel (Lösungsvermittler, Emulgatoren, Dispersionsmittel und Netzmittel), Stabilisatoren, Haftmittel, Aerosol-Treibgase, Synergisten etc.

Beispiele für Lösungsmittel sind Wasser sowie organische Lösungsmittel, z.B. Kohlenwasserstoffe [wie n-Hexan, Petroläther, Schwerbenzin, Erdölfraktionen (Paraffinwachs, Kerosin, Gasöl, Mittelöl, Schweröl etc.), Benzol, Toluol, Xylol etc.], chlorierte Kohlenwasserstoffe [wie Methylenchlorid, Kohlenstofftetrachlorid, Trichlorethylen, Ethylenchlorid, Ethylendibromid, Chlorbenzol, Chloroform etc.], Alkohole [wie Methylalkohol, Ethylalkohol, Propylalkohol, Ethylenglycol etc.], Äther [wie Ethyläther, Ethylenoxid, Dioxan etc.], Alkohol-Äther [wie Ethylenglycol-Monomethyläther etc.], Ketone [wie Aceton, Isophoron etc.], Ester [wie Ethylacetat, Amylacetat etc.], Amide [wie Dimethylformamid, Dimethylacetamid etc.], Sulfoxide [wie Dimethylsulfoxid etc.] und dergleichen.

Beispiele für Füllstoffe oder Träger sind: anorganische pulvrige Feststoffe, z.B. Schwefel, gelöschter Kalk, Magnesiumkalk, Gips, Calciumcarbonat, Kieselerde, Perlit, Bimsstein, Calcit, Diatomit, amorphes Siliciumdioxid, Aluminiumoxid, Zeolith, Tonmineralien (beispielsweise Pyrophyllit, Talkum, Montmorillonit, Beidellit, Vermikulit, Kaolinit, Glimmer etc.); pflanzliche pulvrige Feststoffe, z.B. Getreidepulver, Stärke, verarbeitete Stärke, Zucker, Glucose, zerkleinerte Produkte aus Pflanzenstengeln etc.; aus synthetischen Harzen bestehende pulvrige Feststoffe, z.B. Phenolharze, Harnstoffharze, Vinylchloridharze etc. und dergleichen.

Beispiele für oberflächenaktive Mittel sind: anionische oberflächenaktive Mittel wie Alkylsulfate (z.B. Natriumlaurylsulfat), Arylsulfonsäuren (z.B. Alkylarylsulfonat, Natriumalkylnaphthalinsulfonat etc.), Bernsteinsäure-Salze, Polyethylenglycol-Alkylaryläther-Schwefelsäureester-Salze etc.; cationische oberflächenaktive Mittel wie Alkylamine (z.B. Laurylamin, Stearyltrimethylammoniumchlorid, Alkyldimethylbenzylammoniumchlorid etc.), Polyoxyethylen-Alkylamine etc.; nichtionische oberflächenaktive Mittel wie Polyoxyethylenglycoläther (z.B. Polyoxyethylen-Alkylaryläther und dessen Kondensate etc.), Polyoxyethylen-Alkylaryläther und dessen Kondensate etc.), Polyoxyethylenglycolester (z.B. Polyoxyethylen-Fettsäureester etc.), Polyolester (z.B. Polyoxyethylen-Sorbitan-Monolaurat etc.); ampholytische oberflächenaktive Mittel und dergleichen.

Beispiele für andere Hilfsstoffe sind Stabilisatoren, Haftmittel [z.B. landwirtschaftliche Seifen, Caseinkalk, Natriumalginat, Polyvinylalkohol (PVA), Klebstoffe auf Vinylacetat-Basis, Acryl-Klebstoffe etc.], wirkungsverlängernde Mittel, Dispersionsstabilisatoren [z.B. Casein, Tragant, Carboxylmethylcellulose (CMC), Polyvinylalkohol (PVA) etc.], Synergisten und dergleichen.

Die Verbindungen gemäss der vorliegenden Erfindung können wie bei der Herstellung von Agrochemikalien üblich zu verschiedenenartigen Präparaten verarbeitet werden. Beispiele für solche Präparate sind Emulsionen, Öllösungen, netzbare Pulver, wässrige Lösungen, Suspensionen, Pulver, Granulat, pulvrige Gemische, Kapseln etc.

Die herbiziden Mittel gemäss der vorliegenden Erfindung können die vorgenannten Wirkstoffe in Mengen von 0,001 bis 100 Gewichtsprozent, vorzugsweise von 0,05–95 Gewichtsprozent, enthalten.

Für den praktischen Gebrauch liegt der Gehalt der Wirkstoffe in den verschiedenen Zubereitungen und gebrauchsfertigen Präparaten im allgemeinen im Bereich von 0,01 bis 95 Gewichtsprozent, vorzugsweise von 0,05–60 Gewichtsprozent. Der Gehalt dieser Wirkstoffe kann je nach Art des Präparats, nach Anwendungsmethode, -Zweck, -Zeit und -Ort, der Schwere des Unkrautwachstums etc. variiert werden.

Die Verbindungen gemäss der vorliegenden Erfindung können, falls weiter erforderlich ist, in Kombination mit anderen Agrochemikalien, wie Insektiziden, Fungiziden, Akariziden, Nematoziden, Viroziden, Herbiziden, Pflanzenwachstums-Regulatoren, Lockstoffen [z.B. organischen Phosphat-Verbindungen, Carbamat-Verbindungen, Dithio- (oder-thiol) carbamat-Verbindungen, organischen Chlorverbindungen, Dinitro-Verbindungen, organischen Schwefel- oder Metallverbindungen, Antibiotika, substituierte Diphenyläther-Verbindungen, Harnstoff-Verbindungen, Triazin-Verbindungen etc.], oder/und Düngemittel und dergleichen verwendet werden.

Insbesondere können durch Hinzufügen geeigneter Mengen anderer Wirkstoffe zu den Verbindungen gemäss der vorliegenden Erfindung ein breiteres herbizides Spektrum und eine treffsichere Bekämpfungswirkung erzielt werden, und durch das Vermischen ist auch ein synergistischer Effekt zu erwarten. Beispiele für solche anderen aktiven Wirkstoffe sind folgende:

2-Chloro-2′,6′-diethyl-N-(butoxymethyl)-acetanilid,
N-(O,O-Dipropyl-diethylphosphorylacetyl)-2-methylpiperidin,
S-(4-Chlorobenzyl)-N,N-diethylthiol-Carbamat,
S-Ethyl-N,N-hexamethylenthiol-carbamat,
O-Methyl-O-(2-nitro-p-tolyl)-N-isopropylphosphoramid-thioat,

O-Ethyl-O-(2-nitro-5-methylphenyl)-N-sec-butylphosphoramid-thioat,
3,4-Dimethyl-2,6-dinitro-N-1-ethylpropylanilid,
α,α,α-Trifluoro-2,6-dinitro-N,N-dipropyl-p-toluidin,
2-Chloro-2′,6′-diethyl-N-(n-propoxyethyl)-acetanilid,
4,5-Dichloro-1,3-thiazol-2-yloxyessigsäure-N-isopropyl-N-ethoxyethoxyamid,
5-Ethyl-1,3,4-thiadiazol-2-yloxyessigsäure-1′,2′,3′,4′-tetrahydrochinolid,
Benzothiazol-2-yloxyessigsäure-N,N-diallylamid,
Benzoxazol-2-yloxyessigsäure-N-sec-butyl-N-methylamid,
Benzoxazol-2-yloxyessigsäure-N-cyclohexyl-N-methylamid,
Benzothiazol-2-yloxyessigsäure-N-methyl-N-(1-methylpropargyl)amid,
Benzoxazol-2-yloxyessigsäure-N-benzyl-N-propargylamid,
Benzothiazol-2-yloxyessigsäure-2′-ethylpiperidid,
Benzothiazol-2-yloxyessigsäure-2′,4′-dimethylpiperidid,
Benzoxazol-2-yloxyessigsäure-2′4′6′-trimethylpiperidid,
Benzoxazol-2-yloxyessigsäure-hexamethylenimid,
Benzothiazol-2-yloxyessigsäure-perhydroindolid,
Benzoxazol-2-yloxyessigsäure-perhydroindolid,
Benzothiazol-2-yloxyessigsäure-1′,2′,3′,4′-tetrahydrochinolid,
Benzoxazol-2-yloxyessigsäure-2′methyl-1′,2′,3′,4′-tetrahydrochinolid,
Benzoxazol-2-yloxyessigsäure-N-methylanilid,
Benzothiazol-2-yloxyessigsäure-N-methylanilid,
Benzoxazol-2-yloxyessigsäure-N-ethylanilid,
Benzoxazol-2-yloxyessigsäure-N-propylanilid,
Benzoxazol-2-yloxyessigsäure-N-isopropylanilid,
Benzothiazol-2-yloxyessigsäure-N-methyl-N-2′-methoxyanilid,
Benzoxazol-2-yloxyessigsäure-N-methyl-N-2′-methoxyanilid,
Benzoxazol-2-yloxyessigsäure-N-methyl-N-2′-trifluoromethylanilid,
Benzothiazol-2-yloxyessigsäure-N-methyl-N-2′-chloranilid,
Benzoxazol-2-yloxyessigsäure-N-methyl-N-2′-chloranilid,
Benzothiazol-2-yloxyessigsäure-N-methyl-N-2′-fluoroanilid,
Benzoxazol-2-yloxyessigsäure-N-methyl-N-2′-fluoroanilid,
Benzothiazol-2-yloxyessigsäure-N-methyl-N-3′-methylanilid,
Benzoxazol-2-yloxyessigsäure-N-methyl-N-3′-methylanilid,
Benzothiazol-2-yloxyessigsäure-N-methyl-N-3′-methoxyanilid,
Benzoxazol-2-yloxyessigsäure-N-methyl-N-3′-methoxyanilid,
Benzothiazol-2-yloxyessigsäure-N-methyl-N-3′-isopropoxyanilid,
Benzoxazol-2-yloxyessigsäure-N-methyl-N-3′-isopropoxyanilid,
Benzothiazol-2-yloxyessigsäure-N-methyl-3′-trifluoromethylanilid,
Benzoxazol-2-yloxyessigsäure-N-methyl-N-3′-trifluoromethylanilid,
Benzothiazol-2-yloxyessigsäure-N-methyl-N-3′-chloroanilid,
Benzoxazol-2-yloxyessigsäure-N-methyl-N-3′-chloranilid,
Benzothiazol-2-yloxyessigsäure-N-methyl-N-3′-fluoroanilid,
Benzoxazol-2-yloxyessigsäure-N-methyl-N-3′-fluoroanilid,
Benzothiazol-2-yloxyessigsäure-N-methyl-N-3′-bromoanilid,
Benzoxazol-2-yloxyessigsäure-N-methyl-N-3′-bromoanilid,
Benzoxazol-2-yloxyessigsäure-N-4′-methylanilid,
Benzoxazol-2-yloxyessigsäure-N-methyl-N-4′-methoxyanilid,
Benzoxazol-2-yloxyessigsäure-N-methyl-N-4′-fluoroanilid,
Benzoxazol-2-yloxyessigsäure-N-methyl-N-2′,3′-dimethylanilid,
Benzoxazol-2-yloxyessigsäure-N-methyl-N-2′,3′-dichloroanilid,
Benzoxazol-2-yloxyessigsäure-N-methyl-N-4′-chloro-2′-methylanilid,
Benzothiazol-2-yloxyessigsäure-N-methyl-N-2′,5′-dichloranilid,
Benzoxazol-2-yloxyessigsäure-N-methyl-N-2′,5′-dichloroanilid,
Benzothiazol-2-yloxyessigsäure-N-methyl-N-3′,5′-dimethlanilid,
Benzoxazol-2-yloxyessigsäure-N-methyl-N-3′,5′-dimethylanilid,
Benzoxazol-2-yloxyessigsäure-N-methyl-N-3′,5′-di-trifluoromethylanilid,
Benzoxazol-2-yloxyessigsäure-N-methyl-N-5′-indanylamid,
Benzothiazol-2-yloxyessigsäure-N-methyl-N-3′-ethylanilid,
Benzoxazol-2-yloxyessigsäure-N-methyl-N-3′-ethylanilid,
Benzothiazol-2-yloxyessigsäure-N-isopropylanilid und dergleichen.

Die verschiedenen Zubereitungen und gebrauchsfertigen Präparate, die die vorgenannten Wirkstoffe gemäss der vorliegenden Erfindung enthalten, können mit Hilfe der bei der Anwendung von Agrochemikalien allgemein gebräuchlichen Methoden zum Einsatz gebracht werden, beispielsweise durch Versprühen [wie Lösungsspritzen, Vernebeln, Zerstäuben, Stäuben, Streuen von Granalien, Wasseroberflächenbehandlung, Giessen etc.], Bodenbehandlung [wie Einarbeitung in den Boden, Sprengen etc.] und so weiter. Sie können ebenfalls mit Hifle des sogenannten ULV-Sprühverfahrens (ultra-low-volume spraying method) zur Anwendung gebracht werden, bei der die Formulierung aus 100 Gewichtsprozent des Wirkstoffes oder der Wirkstoffe bestehen kann. Die Dosierung pro Einheitsfläche beträgt etwa 0,1 bis 3 kg/ha, vorzugsweise

0,2 bis 1 kg/ha, berechnet für den Wirkstoff. In besonderen Fällen ist es jedoch möglich oder sogar notwendig, eine höhere oder niedrigere Aufwandsmenge anzuwenden, als dem vorgenannten Bereich entspricht.

Die Erfindung umfasst ferner ein herbizides Mittel, das eine Verbindung der vorgenannten Formel (I) und ein Verdünnungsmittel (ein Lösungsmittel und/oder einen Füllstoff und/oder einen Träger) und/oder ein oberflächenaktives Mittel sowie, falls weiter erforderlich, z.B. einen Stabilisator, ein Haftmittel und einen Synergisten enthält.

Die Erfindung umfasst ferner ein Verfahren zur Unkrautbekämpfung, bei dem die Verbindung der Formel (I) entweder für sich allein oder in Kombination mit einem Verdünnungsmittel (einem Lösungsmittel und/oder einem Füllstoff und/oder einem Träger) und/oder einem oberflächenaktiven Mittel und, falls weiter erforderlich, z.B. einem Stabilisator, einem Haftmittel und einem Synergisten auf Unkräuter oder deren Lebensraum aufgebracht wird.

Die Ausführungsformen der vorliegenden Erfindung werden durch die folgenden Beispiele näher erläutert, jedoch sollte die Erfindung nicht auf diese beschränkt werden.

Beispiel 1 (netzbares Pulver)

15 Teile der Verbindung Nr. 1 der vorliegenden Erfindung, 80 Teile einer 1:5-Mischung aus pulvrigem Diatomit und pulvrigem Ton, 2 Teile Natriumalkylbenzolsulfonat und 3 Teile eines Kondensats aus Natriumalkylnaphthalinsulfonat und Formalin wurden vermahlen und unter Bildung eines netzbaren Pulvers miteinander vermischt. Dieses wird mit Wasser verdünnt und auf die Unkräuter und/oder deren Lebensraum aufgesprüht.

Beispiel 2 (Emulsion)

30 Teile der Verbindung Nr. 2 der Erfindung, 55 Teile Xylol, 8 Teile Polyoxyethylen-Alkylphenyläther und 7 Teile Calcium-Alkylbenzolsulfonat wurden unter Rühren miteinander zu einer Emulsion vermischt. Diese wird mit Wasser verdünnt und auf die Unkräuter und/oder deren Lebensraum aufgebracht.

Beispiel 3 (Staub)

2 Teile der Verbindung Nr. 3 der Erfindung und 98 Teile Tonpulver wurden vermahlen und unter Bildung eines Staubes miteinander vermischt. Dieser wird auf Unkräuter und/oder deren Lebensraum aufgestäubt.

Beispiel 4 (Staub)

1,5 Teile der Verbindung Nr. 4 der Erfindung, 0,5 Teile Isopropyl-Hydrogenphosphat (PAP) und 98 Teile Tonpulver wurden vermahlen und unter Bildung eines Staubes miteinander vermischt. Dieser wird auf die Unkräuter und/oder deren Lebensraum aufgestäubt.

Beispiel 5 (Granulat)

10 Teile der Verbindung Nr. 5 der Erfindung, 30 Teile Bentonit (Montmorillonit), 58 Teile Talkum und 2 Teile Ligninsulfonat wurden miteinander vermischt, und 25 Teile Wasser wurden zu dem Gemisch hinzugefügt und innig mit diesem vermischt. Die erhaltene Mischung wurde mittels eines Extrudergranulators zu Granalien einer Korngrösse von 0,42 bis 2,0 mm (10–40 mesh) pelletisiert und dann unter Bildung eines Granulats bei 40–50°C getrocknet. Das Granulat wird auf Unkräuter und/oder deren Lebensraum aufgestreut.

Beispiel 6 (Granulat)

95 Teile Tonmineral-Teilchen mit einer Teilchengrösse-Verteilung von 0,2–2 mm wurden in einen Drehmischer gegeben, gleichmässig mit 5 Teilen der Verbindung Nr. 6 der Erfindung aufgelöst in einem organischen Lösungsmittel, durch Versprühen dieser Lösung benetzt und unter Bildung eines Granulats bei 40–50°C getrocknet. Das Granulat wird auf die Unkräuter und/oder deren Lebensraum aufgestreut.

Im Vergleich zu bekannten Wirkstoffen mit ähnlicher Struktur und Aktivität sind die neuartigen Verbindungen gemäss der vorliegenden Erfindung dadurch gekennzeichnet, dass sie eine wesentlich verbeserte Wirkung zeigen und keine oder nur eine sehr geringe Toxizität gegenüber Warmblütern aufweisen, und aus diesem Grunde sind die neuen Verbindungen sehr wertvoll.

Die hervorragenden Vorteile und bedeutsamen Wirkungen der Wirkstoffe gemäss der vorliegenden Erfindung können aus den folgenden Ergebnissen eines Tests ersehen werden, in denen diese Verbindungen gegen verschiedene Unkräuter angewandt wurden.

Test-Beispiel 1

Test zur Behandlung von Stengeln und Blättern sowie des Bodens unter Bewässerungsbedingungen gegen in Wasser wachsende Reis-Unkräuter (Pot test):

Herstellung der den Wirkstoff enthaltenden Präparate:

Ein Präparat des speziellen Wirkstoffs kann durch Vermischen eines Gewichtsteils des Wirkstoffs, 5 Gewichtsteilen Aceton als Träger und 1 Gewichtsteil Emulgator unter Bildung einer Emulsion hergestellt werden. Eine bestimmte Menge dieses Präparats wird dann mit Wasser verdünnt.

Test-Verfahren:

Reis-Kulturboden wurde in Wagner-Töpfe von 2 $dm^2$ Fläche gefüllt, und Reis-Setzlinge (Sortenbezeichnung: Kinmaze) im 2–3-Blattstadium (Pflanzenhöhe etwa 10 cm) wurden umgepflanzt, jeweils 2 Setzlinge pro Topf. Dann wurden Samen von Echinochloa crus-galli Beauv. var., Cyperus iria L., Monochoria vaginalis Presl., Scirpus juncoides Roxburgh var. sowie Bruchstücke breitblättriger Unkräuter von Eleocharis acicularis L. und Wurzeln von Cyperus serotinus Rottboell und Sagittaria pygmaea Miq. inokuliert und Feuchtbedingungen aufrechterhalten. Nachdem Echinochloa crus-galli Beauv. var. etwa bis zum 2-Blattstadium (7–9 Tage nach dem Inokulieren) gewachsen war, wurde Wasser bis zu einer Tiefe von etwa 6 cm eingefüllt und eine vorher festgelegte Menge jeder der Verbindungen gemäss der Erfindung in Form einer Emulsion durch Pipettieren dem Wasser zugesetzt. Das Wasser wurde mit einer Geschwindigkeit von 2–3 cm Tiefe pro Tag während zweier Tage nach der Behandlung abgezogen, und danach wurde die Wassertiefe in den Töpfen bei etwa 3 cm gehalten. Vier Wochen nach der chemischen Behandlung wurde die herbizide Wirkung und der Grad der Phytotoxizität auf einer Skala von 0–5 nach dem folgenden Massstab bewertet.

Die Bewertung der Wirkung erfolgt auf der Basis der Unkrautvernichtungsrate im Verhältnis zu derjenigen einer unbehandelten Fläche wie folgt:

5: 95% oder höher (Tod)
4: 80% (einschl.) - 95% (ausschl.)
3: 50% (einschl.) - 80% (ausschl.)
2: 30% (einschl.) - 50% (ausschl.)
1: 10% (einschl.) - 30% (ausschl.)
0: weniger als 10% (keine Wirkung).

Die Bewertung der Phytotoxizität gegenüber Reispflanzen wird aufgrund der Phytotoxizität relativ zu derjenigen auf unbehandelten Flächen vorgenommen und durch folgende Wertzahlen ausgedrückt:

5: 90% oder höher (Ausrottung)
4: 50% (einschl.) - 90% (ausschl.)
3: 30% (einschl.) - 50% (ausschl.)
2: 10% (einschl.) - 30% (ausschl.)
1: wengier als 10%, jedoch nicht 0
0: 0% (keine Phytotoxizität).

Die Ergebnisse sind in Tabelle 1 aufgeführt.

Tabelle 1

| Verbindung Nr. | Aufwandmenge des Wirkstoffes kg/ha | Herbizide Wirkung Unkräuter A | B | C | D | E | F | G | H | Phyto-toxizität Reis |
|---|---|---|---|---|---|---|---|---|---|---|
| 1 | 0,2 | 5 | 5 | 5 | 5 | 5 | 5 | 5 | 5 | 0 |
| 2 | 0,2 | 5 | 5 | 5 | 5 | 5 | 5 | 5 | 5 | 0 |
| 3 | 0,2 | 5 | 5 | 5 | 5 | 5 | 5 | 5 | 5 | 0 |
| 4 | 0,2 | 5 | 5 | 5 | 5 | 5 | 5 | 5 | 5 | 0 |
| 5 | 0,2 | 5 | 5 | 5 | 5 | 5 | 5 | 5 | 5 | 0 |
| 6 | 0,2 | 5 | 5 | 5 | 5 | 5 | 5 | 5 | 5 | 0 |
| 7 | 0,2 | 5 | 5 | 5 | 5 | 5 | 5 | 5 | 5 | 0 |
| 8 | 0,2 | 5 | 5 | 5 | 5 | 5 | 5 | 5 | 5 | 0 |
| Vergl.-Verb. IV-1 | 0,2 | 5 | 5 | 5 | 5 | 5 | 5 | 3 | 5 | 4 |

Anmerkungen

1. Die Nummer der Verbindung entspricht derjenigen, die in den folgenden Herstellungsbeispielen und der nachstehenden Tabelle 2 angegeben ist.

2. Die Symbole A, B, C, D, E, F, G und H stehen für die folgenden Unkräuter:

A: Echinochloa crus-galli Beauv. var.,
B: Eleocharis acicularis L.,
C: Cyperus iria L.,
D: Scirpus juncoides Roxburgh var.,
E: Monochoria vaginalis Presl.,
F: Breitblättrige Unkräuter (wie Lindernia Procumbens Philcox, Rotala indica Koehne, Elatine triandra Schk., etc.),
G: Cyperus serotinus Rotboell,
H: Sagittaria pygmaea Miq.

3. Die Vergleichsverbindung IV-1 ist N-2-Chlorphenylsulfonyl-N′-(4-methoxy-6-methyl-1,3,5-triazin-2-yl)harnstoff der Formel

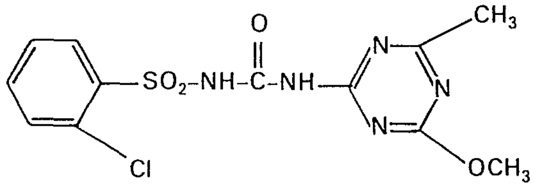

(die in der japanischen Offenlegungsschrift 52-122 384 beschriebene Verbindung).

Das Verfahren zur Herstellung der Verbindungen gemäss der vorliegenden Erfindung wird durch die folgenden Herstellungsbeispiele ausführlicher beschrieben.

Herstellungsbeispiel 1

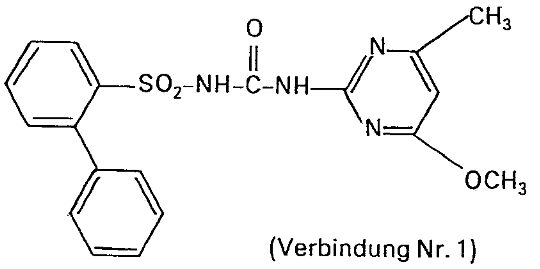

(Verbindung Nr. 1)

13,9 g 2-Amino-4-methoxy-6-methylpyrimidin werden in 150 ml trockenem Dichloromethan aufgelöst. Zu dieser Lösung wird im Verlauf einer Stunde tropfenweise eine Lösung von 28,5 g 2-Biphenylylsulfonylisocyanat in 40 ml Toluol hinzugefügt. Während der Zugabe wird die Innentemperatur auf Raumtemperatur gehalten. Nach Beendigung der Zugabe wird die Mischung zur Vervollständigung der Reaktion 10 Stunden bei Raumtemperatur gerührt. Nach Beendigung der Reaktion wird die Mischung durch Abdampfen des Dichloromethans unter vermindertem Druck etwa auf die Hälfte ihres Volumens eingeengt, und die dabei gebildeten farblosen Kristalle werden abfiltriert. Die Kristalle werden dann mit einer kleinen Menge Äther gewaschen und getrocknet, wonach 34,6 g des gewünschten Produkts, N-2-Biphenylylsulfonyl-N'-(4-methoxy-6-methylpyrimidin-2-yl)-harnstoff, Schmp. 199–202°C, erhalten werden.

Herstellungsbeispiel 2

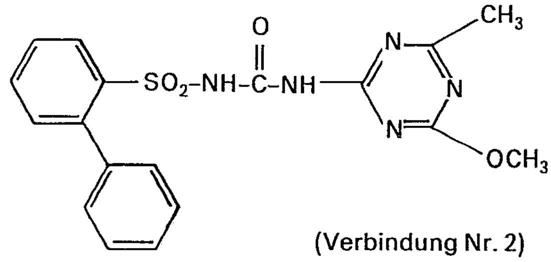

(Verbindung Nr. 2)

14,0 g 2-Amino-4-methoxy-6-methyl-1,3,5-triazin werden in 100 ml trockenem Acetonitril suspendiert und danach 0,1 g 1,4-Diazabicyclo [2.2.2]octan dazugegeben. Zu dieser Mischung wird im Verlauf einer Stunde tropfenweise eine Lösung von 28,5 g 2-Biphenylylsulfonylisocyanat in 30 ml Xylol hinzugefügt. Während der Zugabe wird die Reaktion von einer leichten Wärmeentwicklung begleitet, und deshalb wird erforderlichenfalls gekühlt. Nach der Zugabe wird die Reaktion 5 Stunden bei Raumtemperatur und anschliessend 5 Stunden bei 40°C fortgesetzt. Man lässt den Inhalt auf Raumtemperatur abkühlen und filtriert die ausgefallenen farblosen Kristalle ab, wäscht sie mit Äther und trocknet, wonach 33,9 g des gewünschten Produkts, N-2-Biphenylylsulfonyl-N'-(4-methoxy-6-methyl-triazin-2-yl)-harnstoff, Schmp. 190–193°C, erhalten werden.

Die Verbindungen der vorliegenden Erfindung, die in ähnlicher Weise wie die vorbeschriebenen hergestellt werden, sind in Tabelle 2 aufgeführt.

Tabelle 2

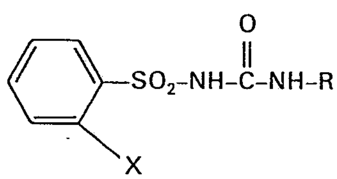

| Verbindung Nr. | X | R | Physik. Konstante Schmp. (°C) |
|---|---|---|---|
| 3 | 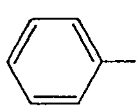 | 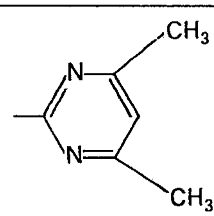 | 203–208 |
| 4 | 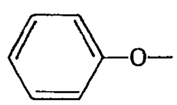 | 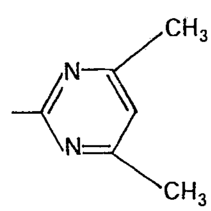 | 178–180 |
| 5 | 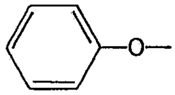 | 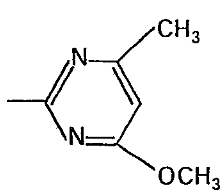 | 196–200 |

Tabelle 2 (Fortsetzung)

| Verbindung Nr. | X | R | Physik. Konstante Schmp. (°C) |
|---|---|---|---|
| 6 | 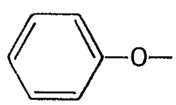 | 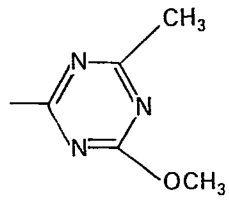 | 185–190 |
| 7 | 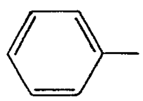 | 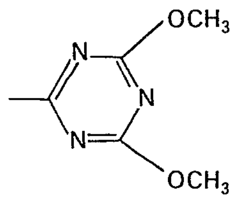 | 175–180 |
| 8 | 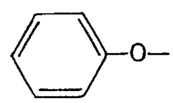 | 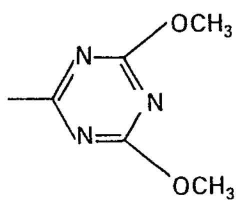 | 160–165 |

**Patentansprüche für die Vertragsstaaten BE CH DE FR GB IT LI NL SE**

1. Substituiertes Phenylsulfonylharnstoff-Derivat der Formel

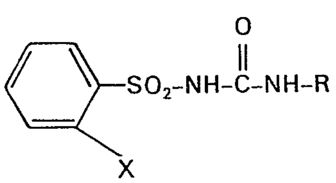 (I)

in der X für eine Phenoxy-Gruppe und R für eine der Gruppen

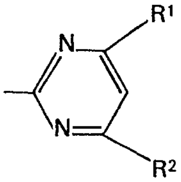 oder 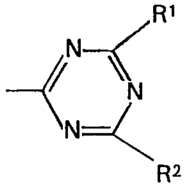 ;

stehen, worin $R^1$ und $R^2$ eine Methyl-Gruppe oder eine Methoxy-Gruppe bezeichnet.

2. N-2-Phenoxyphenylsulfonyl-N′-(4-methoxy-6-methyl-pyrimidin-2-yl)-harnstoff nach Anspruch 1 der Formel

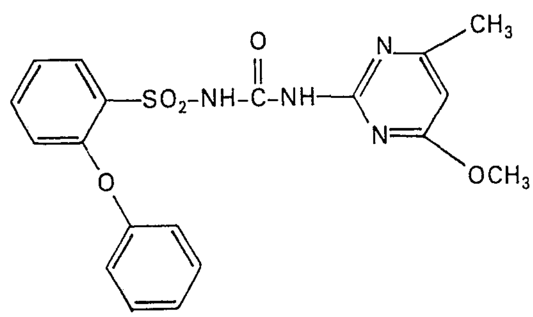

3. Verfahren zur Herstellung eines substituierten Phenylsulfonylharnstoff-Derivats der Formel

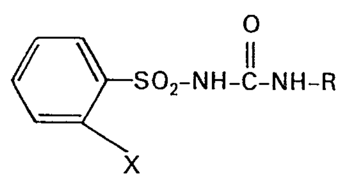 (I)

in der X für eine Phenoxy-Gruppe und R für eine der Gruppen

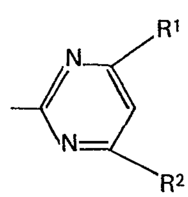 oder 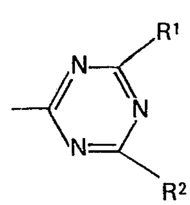 ;

stehen, worin $R^1$ und $R^2$ eine Methyl-Gruppe oder

eine Methoxy-Gruppe bezeichnen, dadurch gekennzeichnet, dass eine Verbindung der Formel

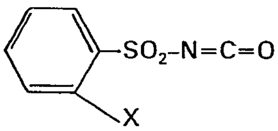
(II)

in der X die vorstehend genannte Bedeutung hat, mit einer Verbindung der Formel

$NH_2$-R (III)

in der R die vorstehend genannte Bedeutung hat, gegebenenfalls in Anwesenheit eines Verdünnungsmittels, umgesetzt wird.

4. Herbizides Mittel, gekennzeichnet durch einen Gehalt an mindestens einem substituierten Phenylsulfonylharnstoff-Derivat der Formel (I) gemäss Anspruch 1.

5. Verwendung von substituierten Phenylharnstoff-Derivaten der Formel (I) gemäss Anspruch 1 zur Bekämpfung von Unkräutern.

6. Verfahren zur Herstellung von herbiziden Mitteln, dadurch gekennzeichnet, dass man substituierte Phenylsulfonylharnstoffe der Formel (I) gemäss Anspruch 1 mit Streckmitteln und/oder oberflächenaktiven Mitteln vermischt.

**Patentansprüche für den Vertragsstaat AT**

1. Verfahren zur Herstellung eines substituierten Phenylsulfonylharnstoff-Derivats der Formel

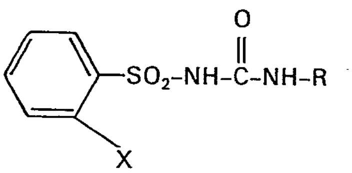
(I)

in der X für eine Phenyl-Gruppe oder eine Phenoxy-Gruppe und R für eine der Gruppen

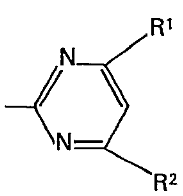
oder
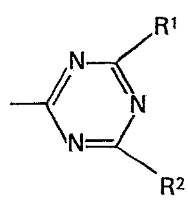
;

stehen, worin $R^1$ und $R^2$ eine Methyl-Gruppe oder eine Methoxy-Gruppe bezeichnen, dadurch gekennzeichnet, dass eine Verbindung der Formel

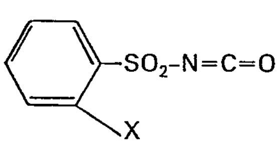
(II)

in der X die vorstehend genannte Bedeutung hat, mit einer Verbindung der Formel

$NH_2$-R (III)

in der R die vorstehend genannte Bedeutung hat, gegebenenfalls in Anwesenheit eines Verdünnungsmittels, umgesetzt wird.

2. Herbizides Mittel, gekennzeichnet durch einen Gehalt an mindestens einem substituierten Phenylsulfonylharnstoff-Derivat der Formel (I) gemäss Anspruch 1.

3. Verwendung von substituierten Phenylharnstoff-Derivaten der Formel (I) gemäss Anspruch 1 zur Bekämpfung von Unkräutern.

4. Verfahren zur Herstellung von herbiziden Mitteln, dadurch gekennzeichnet, dass man substituierte Phenylsulfonylharnstoffe der Formel (I) gemäss Anspruch 1 mit Streckmitteln und/oder oberflächenaktiven Mitteln vermischt.

**Claims for the Contracting States: BE CH DE FR GB IT LI NL SE**

1. Substituted phenylsulphonylurea derivative of the formula

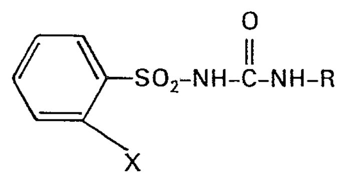
(I)

in which
X represents a phenoxy group and
R represents one of the groups

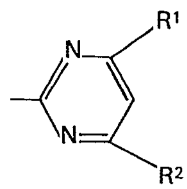
or
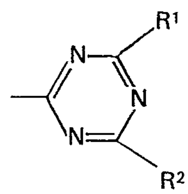
;

wherein
$R^1$ and $R^2$ designates a methyl group or a methoxy group.

2. N-2-Phenoxyphenylsulphonyl-N'-(4-methoxy-6-methyl-pyrimidin-2-yl)-urea according to Claim 1 of the formula

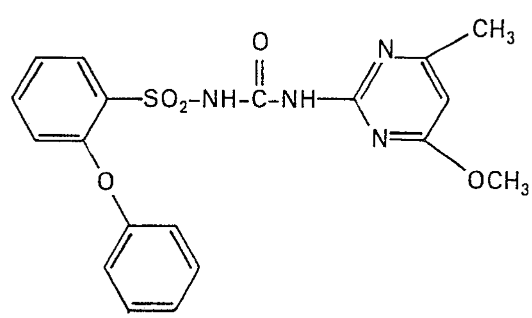

3. Process for the preparation of a substituted phenylsulphonylurea derivative of the formula

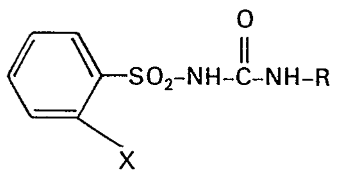

(I)

in which
X represents a phenoxy group and
R represents one of the groups

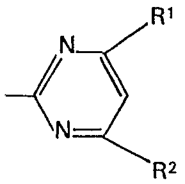

or

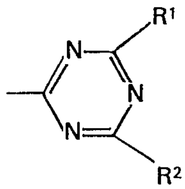

;

wherein
$R^1$ and $R^2$ designate a methyl group or a methoxy group, characterised in that a compound of the formula

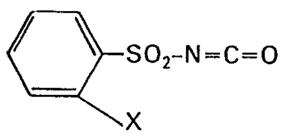

(II)

in which
X has the abovementioned meaning, is reacted with a compound of the formula

$NH_2$-R (III)

in which
R has the abovementioned meaning, if appropriate in the presence of a diluent.

4. Herbicidal agent, characterised in that it contains at least one substituted phenylsulphonylurea derivative fo the formula (I) according to Claim 1.

5. Use of substituted phenylurea derivatives of the formula (I) according to Claim 1 for combating weeds.

6. Process for the preparation of herbicidal agents, characterised in that substituted phenylsulphonylureas of the formula (I) according to Claim 1 are mixed with extenders and/or surface-active agents.

**Claims for the Contracting State: Austria**

1. Process for the preparation of a substituted phenylsulphonylurea derivative of the formula

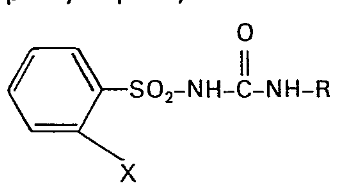

(I)

in which
X represents a phenyl group or a phenoxy group and
R represents one of the groups

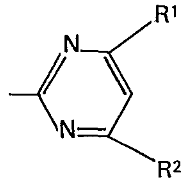

or

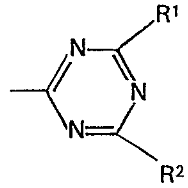

;

wherein
$R^1$ and $R^2$ designate a methyl group or a methoxy group, characterised in that a compound of the formula

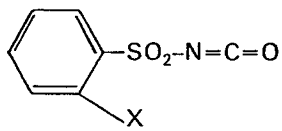

(II)

in which
X has the abovementioned meaning, is reacted with a compound of the formula

$NH_2$-R (III)

in which
R has the abovementioned meaning, if appropriate in the presence of a diluent.

2. Herbicidal agent, characterised in that it contains at least one substituted phenylsulphonylurea derivative fo the formula (I) according to Claim 1.

3. Use of substituted phenylurea derivatives of the formula (I) according to Claim 1 for combating weeds.

4. Process for the preparation of herbicidal agents, characterised in that substituted phenylsulphonylureas of the formula (I) according to Claim 1 are mixed with extenders and/or surface-active agents.

**Revendications pour les États contractants: BE, CH, DE, FR, GB, IT, LI, NL, SE**

1. Derivé substitué de phénylsulfonylurée de formule

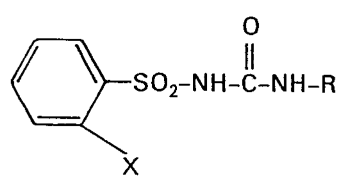

(I)

dans laquelle X représente un groupe phénoxy et R représente l'un des groupes

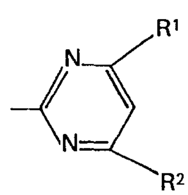

ou

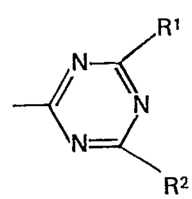

;

où $R^1$ et $R^2$ représentent un groupe méthyle ou un groupe méthoxy.

2. N-2-phénoxyphénylsulfonyl-N′-(4-méthoxy-6-méthylpyrimidine-2-yl)-urée suivant la revendication 1, de formule

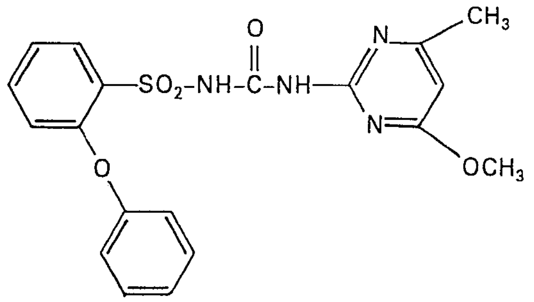

3. Procédé de production d'un dérivé substitué de phénylsulfonylurée de formule

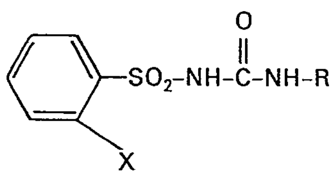
(I)

dans laquelle X est un groupe phénoxy et R est l'un des groupes

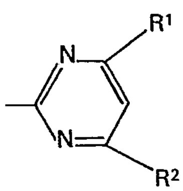
ou
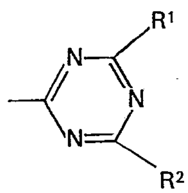
;

où $R^1$ et $R^2$ représentent un groupe méthyle ou un groupe méthoxy, caractérisé en ce qu'on fait réagir un composé de formule

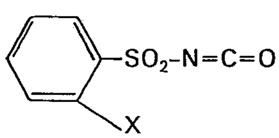
(II)

dans laquelle X a la définition indiquée ci-dessus, avec un composé de formule

$NH_2$-R (III)

dans laquelle R a la définition donnée ci-dessus, le cas échéant en présence d'un diluant.

4. Composition herbicide, caractérisée par une teneur en au moins un dérivé substitué de phénylsulfonylurée de formule (I) suivant la revendication 1.

5. Utilisation de dérivés substitués de phénylurée de formule (I) suivant la revendication 1 pour combattre des mauvaises herbes.

6. Procédé de préparation de compositions herbicides, caractérisé en ce qu'on mélange des phénylsulfonylurées substituées de formule (I) suivant la revendication 1 avec des diluants et/ou des agents tensio-actifs.

**Revendications pour l'État contractant: AT**

1. Procédé de production d'un dérivé substitué de phénylsulfonylurée de formule

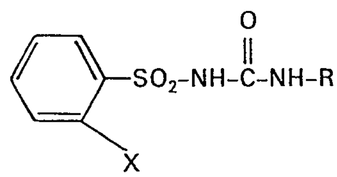
(I)

dans laquelle X est un groupe phényle ou un groupe phénoxy et R représente l'un des groupes

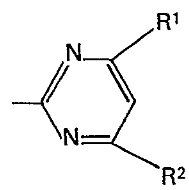
ou
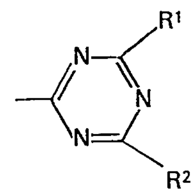
;

où $R^1$ et $R^2$ désignent un groupe méthyle ou un groupe méthoxy, caractérisé en ce qu'on fait réagir un composé de formule

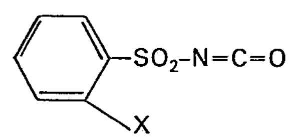
(II)

dans laquelle X a la définition indiquée ci-dessus, avec un composé de formule

$NH_2$-R (III)

dans laquelle R a la définition indiquée ci-dessus, éventuellement en présence d'un diluant.

2. Composition herbicide, caractérisée par une teneur en au moins un dérivé substitué de phénylsulfonylurée de formule (I) suivant la revendication 1.

3. Utilisation de dérivés substitués de phénylurée de formule (I) suivant la revendication 1 pour combattre des mauvaises herbes.

4. Procédé de préparation de compositions herbicides, caractérisé en ce qu'on mélange des phénylsulfonylurées substituées de formule (I) suivant la revendication 1 avec des diluants et/ou des agents tensio-actifs.